# EUROPÄISCHE PATENTSCHRIFT

(11) **EP 2 594 492 B1**
(45) Veröffentlichungstag und Bekanntmachung des Hinweises auf die Patenterteilung: **10.05.2017**
(21) Anmeldenummer: 12193686.8
(22) Anmeldetag: 21.11.2012
(51) Int. Cl.: B65B 55/08, A61L 2/08

(54) **Vorrichtung zum Sterilisieren eines Behältnisses, Getränkeabfüllanlage und/oder Getränkebehälterherstellanlage sowie Verfahren zum Sterilisieren eines ein von einer Innenwandung umgrenzten Volumen aufweisenden Behältnisses**
Device for sterilising a container, drink filling assembly and/or drinks container manufacturing plant and method for sterilising a container having a volume which is limited by internal cladding
Dispositif de stérilisation d'un récipient, installation de remplissage de boissons et/ou installation de fabrication de récipients de boissons ainsi que le procédé de stérilisation d'un récipient contenant des volumes limités par une paroi intérieure

(30) Priorität: 21.11.2011 DE 102011055555
(43) Veröffentlichungstag der Anmeldung: 22.05.2013
(73) Patentinhaber: Krones AG, 93073 Neutraubling (DE)
(72) Erfinder: Knott, Josef, 93073 Neutraubling (DE); Engelhard, Patrick, 93073 Neutraubling (DE); Föll, Eberhard, 93073 Neutraubling (DE)
(74) Vertreter: Bittner, Bernhard

(56) Entgegenhaltungen:
- EP-A1- 2 218 465
- JP-A- 2002 308 229
- US-A1- 2007 283 667

## Beschreibung

Die Erfindung betrifft einerseits eine Vorrichtung zum Sterilisieren eines Behältnisses mit einer Erzeugungseinrichtung zum Erzeugen von Ladungsträgern und mit einer in das Behältnis einbringbaren Leiteinrichtung zum Leiten der Ladungsträger auf eine Innenwandung des Behältnisses, wobei eine Mündung des Behältnisses mittels der Trageeinrichtung zum Tragen des Behältnisses gegenüber der Leiteinrichtung positionierbar ist.

Die Erfindung betrifft andererseits eine Getränkeabfüllanlage und/oder eine Getränkebehälterherstellanlage mit einer Vorrichtung zum Umformen von Kunststoffvorformlingen zu Kunststoffbehältnissen.

Des Weiteren betrifft die Erfindung ein Verfahren zum Sterilisieren eines ein von einer Innenwandung umgrenzten Volumen aufweisenden Behältnisses, bei welchem zum Sterilisieren des Behältnisses Ladungsträger erzeugt und die erzeugten Ladungsträger aus einer Austrittsöffnung einer Leiteinrichtung zum Leiten der Ladungsträger heraus in Richtung des Behältnisses beschleunigt werden, um das Behältnis mit Hilfe der Ladungsträger zumindest teilweise zu sterilisieren, wobei das Behältnis der Leiteinrichtung mittels einer translatorischen Relativbewegung und insbesondere Zustellbewegung des Behältnisses zugestellt wird. Im Folgenden wird die Erfindung unter der Maßgabe beschrieben, dass das Behältnis selbst translatorisch bewegt wird. Es wäre jedoch auch möglich, dass die Leiteinrichtung translatorisch bewegt wird und nicht das Behältnis.

Insbesondere gattungsgemäße Vorrichtungen zum Sterilisieren von Behältnissen sind speziell aus der Lebensmittelindustrie bereits gut bekannt und werden dort erfolgreich zur Sterilisation von entsprechenden Behältnissen eingesetzt, bevor diese im weiteren Verlauf mit Lebensmitteln, wie etwa leicht verderblichen Getränken, befüllt werden. In diesem Zusammenhang ist es bekannt, Innenwandungen von Behältnissen mit Dampf oder Wasserstoffperoxid zu sterilisieren. Dies ist jedoch auch nachteilig, da es speziell bei der Behandlung von Kunststoffbehältnissen mit Wasserstoffperoxid zu einer Aufweichung des Kunststoffmaterials kommen kann. Daher ist es aus dem Stand der Technik auch seit langem bekannt, derartige Behältnisse mit Hilfe von Ladungsträgern zu sterilisieren.

Beispielsweise ist aus der Europäischen Patentanmeldung EP 1 982 921 A1 eine gattungsgemäße Vorrichtung zum Sterilisieren von Behältnissen bekannt, welche eine Ladungsträgerquelle zum Erzeugen von Ladungsträgern umfasst, mittels welchen die Behältnisses vorteilhaft auch innen sterilisiert werden können. Hierbei werden die Ladungsträger in einem länglichen Behandlungskopf entlang einer Beschleunigungsstrecke beschleunigt und nach dem Austritt aus dem Behandlungskopf gegen eine Innenwandung des jeweiligen Behältnisses geleitet, wobei der Behandlungskopf vorteilhafter Weise durch einen Mündungsbereich des Behältnisses hindurch geführt und so in das Behältnis eingetaucht werden kann, um die Ladungsträger besonders effektiv an die Innenwandung leiten zu können. Um die Behältnisse auch außen großflächig sterilisieren zu können, ist eine weitere Sterilisationsvorrichtung vorgesehen, an welcher die einzelnen Behältnisse jeweils um ihre Hochachse drehend sukzessiv vorbeigeführt werden.

Ein anderes Sterilisationssystem für PET-Behälter ist aus der internationalen Patentanmeldung WO 2008/129397 A2 bekannt. Bei diesem Sterilisierungssystem wird der zu sterilisierende PET-Behälter für eine Innensterilisation unter einer Sterilisationslanze zwischengeparkt, wobei die Sterilisationslanze durch einen Mündungsbereich hindurch in den PET-Behälter eingetaucht wird, um diesen in seinem Inneren zu sterilisieren. Hierbei ist der PET-Behälter an seinem Behälterhals durch eine Klemmeinrichtung ergriffen und sogleich um seine Hochachse drehbar vor bzw. gegenüber der Sterilisationslanze in Position gehalten. Hierzu weist die Klemmeinrichtung eine entsprechende Rollenmechanik auf. Angetrieben wird der so an der Klemmeinrichtung gelagerte PET-Behälter durch eine rotierbare Hohlstange, in welcher die Sterilisationslanze translatorisch verschiebbar angeordnet ist. Sowohl die Hohlstange als auch die Sterilisationslanze können dem PET-Behälter auch unabhängig voneinander zugestellt werden. Oberhalb des Mündungsbereichs des PET-Behälters ist an der Klemmeinrichtung noch eine profilierte Reflektionsplatte vorgesehen, die zum einen eine Reflektion eines Elektronenstrahls gewährleisten kann, welcher ansonsten am Behälterhals verloren ginge. Zum anderen kann er die Energie des Elektronenstrahls in Röntgenstrahlen konvertieren. Während insbesondere mittels der Sterilisationslanze eine Innensterilisation erzielt wird, erfolgt eine Außensterilisation der PET-Behälters durch eine seitlich des PET-Behälters platzierte weitere Sterilisationsvorrichtung, an welcher der PET-Behälter vorbei führbar ist.

Weiterhin ist beispielsweise auch aus der JP 2002 308 229 eine Vorrichtung und ein Verfahren zur Sterilisation von Behältnissen mittels Elektronenstrahlen bekannt, bei der über elektromagnetische Linsen der Elektronenstrahl derart abgelenkt wird, dass er auch die Außenwandung der Behältnisse trifft.

Es ist Aufgabe der Erfindung gattungsgemäße Sterilisationsvorrichtungen baulich einfacher zu gestalten, sodass sie sich insbesondere platzsparender in eine Getränkeabfüllanlage und/oder eine Getränkebehälterherstellanlage integrieren lassen.

Die Aufgabe der Erfindung wird von einer Vorrichtung zum Sterilisieren eines Behältnisses mit einer Erzeugungseinrichtung zum Erzeugen von Ladungsträgern und mit einer in das Behältnis einbringbaren Leiteinrichtung zum Leiten der Ladungsträger auf eine Innenwandung des Behältnisses, wobei eine Mündung des Behältnisses mittels einer Trageeinrichtung zum Tragen des Behältnisses gegenüber der Leiteinrichtung positionierbar ist, gelöst, und wobei die Vorrichtung eine gegenüber der Leiteinrichtung verlagerbare Umlenkeinrichtung zum Umlenken der aus der Leiteinrichtung austretenden Ladungsträger auf einen außenliegenden Mündungsbereich des Behältnisses aufweist. Vorteilhaft ist die Umlenkeinrichtung auch gegenüber der Trageeinrichtung verlagerbar, beispielsweise in Längsrichtung des Behältnisses verlagerbar.

Dadurch, dass die vorliegende Umlenkvorrichtung vorteilhaft sowohl gegenüber der Leiteinrichtung als auch gegenüber der Trageeinrichtung verlagerbar angeordnet ist, können insbesondere die Leiteinrichtung, die Umlenkeinrichtung und das Behältnis konstruktiv besonders einfach zueinander günstig positioniert werden, um einerseits eine Innensterilisation des Behältnisses und andererseits eine Außensterilisation des Behältnisses zumindest an dem außenliegenden Mündungsbereich zu erzielen.

Es wäre jedoch auch denkbar, dass die Umlenkeinrichtung an die Halteeinrichtung gekoppelt ist, etwa indem sowohl die Halteeinrichtung als auch die Umlenkeinrichtung klammerartig ausgeführt sind.

Die Begrifflichkeit "Erzeugungseinrichtung" beschreibt im Sinne der Erfindung jegliche Einrichtung, mittels welcher zum Sterilisieren geeignete Ladungsträger erzeugt und an der Leiteinrichtung bereitgestellt werden können. Als solche Erzeugungseinrichtungen eignen sich insbesondere sowohl offene als auch geschlossene Elektronenstrahleinheiten, welche beispielsweise aus Röntgenröhren oder ähnlichem bekannt sind. Die Erzeugungseinrichtung kann als punktförmige oder flächige Ladungsträgerquelle und insbesondere Elektronenquelle ausgeführt sein, an welche ein Gehäuse der Leiteinrichtung unmittelbar angebracht ist.

Als "Leiteinrichtung" kommen im Sinne der Erfindung jegliche Einrichtungen in Frage, mit bzw. in welchen sich die erzeugten Ladungsträger auf das zu sterilisierende Behältnis beschleunigen lassen. Hierzu weist die Leiteinrichtung zumindest eine Austrittsöffnung auf, durch welche hindurch die Ladungsträger die Leiteinrichtung verlassen können. Es versteht sich, dass die Austrittsöffnung noch mit Elementen versehen sein kann, mittels welchen die Ladungsträger zusätzlich beeinflusst werden können. Gegebenenfalls kann an der Austrittsöffnung eine Ablenkeinrichtung vorgesehen sein, um die Ladungsträger hinsichtlich ihrer Austrittsrichtung bereits zu beeinflussen. Bei Bedarf können auch mehr als eine derartige Austrittsöffnung an der Leiteinrichtung vorgesehen sein. Vorteilhafter Weise zeichnet sich die Leiteinrichtung durch ein stabförmiges Gehäuse aus, dessen Durchmesser derart gewählt ist, dass es durch eine Mündungsöffnung eines Behältnisses hindurch problemlos in das jeweilige Behältnis eingetaucht werden kann, wie dies aus dem Stand der Technik bereits bekannt ist.

Der Begriff "Ladungsträger" impliziert insbesondere Elektronenstrahlteilchen, wobei auch andere Ladungsträger, wie beispielsweise Ionen, zum Einsatz kommen können.

Der außenliegende Mündungsbereich befindet sich in der Regel oberhalb einer Zugangsöffnung bzw. Mündung des vorliegenden Behältnisses, wobei es sich bei den Behältnissen in erster Linie um Getränkeflaschen handelt, welche vorzugsweise mittels eines Blas- oder Streckblasverfahrens hergestellt sind oder um die Preformen selbst handelt. Der au ßenliegende Mündungsbereich kann noch ein Gewinde für einen Schraubverschluss umfassen. Da von einem nicht sterilisierten außenliegenden Mündungsbereich die Gefahr ausgeht, dass Keime oder dergleichen unbeabsichtigt in das Volumen des Behältnisses gelangen können und dort den Inhalt des Behältnisses kontaminieren, ist es besonders wichtig, dass zumindest in diesem außenliegenden Mündungsbereich eine Sterilisation vorgenommen wird.

Die Trageeinrichtung des Behältnisses kann hierbei ein Haltearmelement mit einer Behältnisaufhängung und/oder mit einem Greifer für die Mündung oder dergleichen umfassen und Teil einer Zustelleinrichtung zum Zustellen des Behältnisses sein. Derartige Trageeinrichtungen sind aus dem Stand der Technik gut bekannt und können baulich einfach zum Einsatz an der vorliegenden Erfindung angepasst werden.

Es versteht sich, dass die vorliegende Umlenkeinrichtung für Ladungsträger von unterschiedlich ausgestalteten Bauteilen oder Bauteilgruppen realisiert werden kann. Um die aus der Leiteinrichtung austretenden Ladungsträger möglichst effektiv auf den außenliegenden Mündungsbereich umlenken zu können, ist es vorteilhaft, wenn die Umlenkeinrichtung zumindest bereichsweise aus einem Material mit einer Dichte besteht, welche höher ist als eine Materialdichte anderer Komponenten der Vorrichtung. Bevorzugt werden hier also Materialien mit einer möglichst hohen Dichte verwendet, denn je höher die Dichte des Materials gewählt ist, desto höher ist auch die erzielbare Reflexion der Ladungsträger an der Umlenkeinrichtung und somit auch die erzielbare Dosis bei der Sterilisation. Hierbei sind auch lediglich Oberflächenbeschichtungen an der Umlenkeinrichtung möglich, um Gewicht einzusparen. Besonders vorteilhaft kann ein Material für die Umlenkeinrichtung oder für die Oberflächenbeschichtung mit einer Dichte von mehr als 9 g/cm³ vorgesehen werden. Idealerweise soll das verwendete Material zudem reinraumtauglich sein.

Um die Umlenkeinrichtung vorteilhaft gegenüber dem Behältnis positionieren zu können, kann es vorteilhaft sein, die Umlenkvorrichtung entlang einer Kurve zu bewegen, um hierdurch Klapp- und/oder Schenkbewegungen zu erhalten.

Eine bevorzugte Ausführungsvariante sieht vor, dass die Umlenkeinrichtung ein konisch zulaufendes Umlenkschirmelement aufweist. Durch das konisch zulaufende Umlenkschirmelement können die aus der Leiteinrichtung austretenden Ladungsträger vorteilhaft außen auf den Mündungsbereich umgelenkt werden.

Vorteilhaft weist die Umlenkeinrichtung wenigstens zeitweise eine Öffnung auf, von der eine Mündung bzw. ein Gewindebereich des Behältnisses umgebbar ist bzw. in Umfangsrichtung aufnehmbar ist. Dabei ist es möglich, dass die Umlenkeinrichtung permanent eine derartige Öffnung aufweist, in die der Mündungsbereich einführbar ist, es wäre jedoch auch denkbar, dass die Umlenkeinrichtung klammerartig um den Mündungsbereich geschlossen wird.

Vorteilhafter Weise ist das Umlenkschirmelement konzentrisch um die Leiteinrichtung anordenbar, insbesondere wenn der Mündungsbereich des Behältnisses zumindest teilweise innerhalb der Umlenkeinrichtung angeordnet ist. Hierdurch können die aus der Leiteinrichtung austretenden Ladungsträger besonders sicher an den gesamten Umfang des außenliegenden Mündungsbereichs umgeleitet werden.

Es ist darüber hinaus vorteilhaft, wenn die Umlenkeinrichtung ein zweigeteiltes Umlenkschirmelement umfasst. Mittels eines derart mehrteilig ausgestalteten Umlenkschirmelementes ergibt sich vorteilhafter Weise eine Vielzahl an Zuführwegen, entlang derer die Umlenkeinrichtung insbesondere der Mündung des Behältnisses zugeführt werden kann.

Ein Sterilisationsvorgang im Sinne der Erfindung kann baulich extrem einfach bewerkstelligt werden, wenn bei in das Behältnis eingebrachter Leiteinrichtung zwischen der Umlenkeinrichtung und der Leiteinrichtung eine Wandung des Mündungsbereiches des Behälters zumindest teilweise angeordnet ist.

Eine besonders bevorzugte Ausführungsvariante sieht des Weiteren vor, dass die Vorrichtung eine Halteeinrichtung für die Umlenkeinrichtung aufweist, an welcher die Umlenkeinrichtung mittels einer Relativbewegung und insbesondere Zustellbewegung (oder Entfernungsbewegung) des Behältnisses gegenüber der Leiteinrichtung translatorisch verlagerbar angeordnet ist und/oder gegenüber welcher die Trageeinrichtung mittels einer Relativbewegung und/oder Zustellbewegung des Behältnisses translatorisch verlagerbar angeordnet ist.

Durch eine derartige Variante kann die vorliegende Vorrichtung zum Sterilisieren hinsichtlich ihres konstruktiven Aufbaus nochmals vereinfacht werden.

An dieser Stelle sei erwähnt, dass die Aufgabe der Erfindung auch von einem Verfahren zum Sterilisieren eines ein von einer Innenwandung umgrenzten Volumen aufweisenden Behältnisses gelöst wird, bei welchem zum Sterilisieren des Behältnisses Ladungsträger erzeugt und die erzeugten Ladungsträger aus einer Austrittsöffnung einer Leiteinrichtung zum Leiten der Ladungsträger heraus in Richtung des Behältnisses beschleunigt werden, um das Behältnis mit Hilfe der Ladungsträger zumindest teilweise zu sterilisieren, wobei das Behältnis der Leiteinrichtung mittels einer translatorischen Relativbewegung bzw. Zustellbewegung und/oder Entfernungsbewegung des Behältnisses gegenüber der Leiteinrichtung zugestellt wird, und wobei sich das Verfahren darüber hinaus dadurch auszeichnet, dass in einem vorgegebenen, z.B. ersten Wegabschnitt der translatorischen Relativbewegung eine Mündung des Behältnisses in eine Umlenkeinrichtung zum Umlenken der aus der Leiteinrichtung austretenden Ladungsträger zumindest teilweise hinein verlagert ist, sodass ein außenliegender Mündungsbereich des Behältnisses in einen Strahlengang der umgelenkten Ladungsträger hinein verlagert und hierdurch mit Hilfe der Ladungsträger sterilisiert wird.

Vorteilhaft wird in einem weiteren Wegabschnitt der translatorischen Relativbewegung die Umlenkeinrichtung gemeinsam mit dem Behältnis derart in Richtung Leiteinrichtung verlagert, dass die Leiteinrichtung durch die Umlenkeinrichtung hindurch in das Volumen des Behältnisses (oder aus diesem Volumen heraus) verlagert wird, sodass die Innenwandung des Behältnisses mit Hilfe der Ladungsträger sterilisiert wird.

Bei diesem vorteilhaften Verfahren wird davon ausgegangen, dass die Sterilisation des Mündungsbereiches insbesondere während einer Zustellbewegung des Behältnisses bzw. vor dem Einführen der Leiteinrichtung (auch als Strahlfinger bezeichnet) in das Behältnis erfolgt. Es wäre jedoch auch denkbar, dass die Sterilisation des Mündungsbereiches erst nach der Sterilisation der Innenwandung erfolgt, insbesondere während eines Herausziehens der Leiteinrichtung aus dem Behältnis oder nach dessen Herausziehen. Bevorzugt findet die Sterilisation des Behältnisses bzw. die Beaufschlagung der Behältniswandungen während der besagten Relativbewegung statt.

Vorteilhafter Weise wird im Verlauf des Sterilisationsprozess speziell die Umlenkeinrichtung mittels einer translatorischen Zustellbewegung gegenüber der vorliegenden Leiteinrichtung verlagert, wodurch sich der Ablauf einer Sterilisation des Behältnisses wesentlich vereinfachen lässt.

Es ist auch denkbar, dass die translatorische Zustellbewegung auch einen rotatorischen Anteil aufweisen kann, wenn dies für eine Zustellung vorteilhaft erscheint.

Vorteilhaft werden die Behältnisse während dieses Sterilisationsvorgangs entlang eines vorgegebenen Transportpfades bewegt. Dieser Transportpfad steht dabei vorteilhaft senkrecht zu einer Richtung der oben beschriebenen Zustell- bzw. Relativbewegung.

Idealerweise wird die translatorische Zustellbewegung mittels einer Hubbewegung umgesetzt, bei welcher das Behältnis mittels eines an sich bekannten Hubmechanismus angehoben und hierbei unterseitig an die Leiteinrichtung heran geführt wird.

Darüber hinaus ist es vorteilhaft, wenn die Vorrichtung eine Linearführungseinheit für die Umlenkeinrichtung mit einem daran translatorisch geführten Schiebeelement zum Verlagern der Umlenkeinrichtung aufweist. Hierdurch kann konstruktiv einfach eine Halteeinrichtung für die Umlenkeinrichtung bereitgestellt werden, mittels welcher insbesondere eine Hubbewegung einer Zustelleinrichtung für das Behältnis baulich sehr einfach auf die Umlenkeinrichtung übertragen werden kann.

Insofern zeichnet sich die Linearführungseinheit durch einen Antrieb aus, welcher die Zustelleinrichtung des Behälters umfasst.

Ist die Linearführungseinheit hierbei vorteilhaft im Wesentlichen parallel und radial neben der Leiteinrichtung und beabstandet von der Trageeinrichtung des Behältnisses angeordnet, können selbst bestehende Vorrichtungen zum Sterilisieren von Behältnissen mit geringem Aufwand nachgerüstet werden.

Alternativ kann die Linearführungseinheit die Trageeinrichtung des Behältnisses umfassen. Hierdurch kann die Vorrichtung zum Sterilisieren nochmals kompakter gebaut werden.

Es ist denkbar, dass die vorliegende Linearführungseinheit konstruktiv sehr unterschiedlich gebaut werden kann. In einem sehr einfachen Fall kann sie eine Führungsstange oder ein Führungsrohr zum Führen der Umlenkeinrichtung umfassen, welches mit der Zustellbewegung fluchtet. Eine bessere Verdrehsicherheit kann gewährleistet werden, wenn eine Doppelführungstange bzw. ein Doppelführungsrohr vorgesehen sind.

Gegebenenfalls kann sich die Linearführungseinheit auch durch eine Kurve auszeichnen, entlang welcher das Schiebeelement verschoben wird, wenn dies vorteilhaft erscheint.

Das Schiebeelement kann bevorzugt als Schiebeschlitten ausgestaltet werden.

Zudem ist es vorteilhaft, wenn die Vorrichtung eine Linearführungseinheit für die Umlenkeinrichtung mit einem daran translatorisch geführten Schiebeelement zum Verlagern der Umlenkeinrichtung aufweist, an welcher die Umlenkeinrichtung bzw. das Schiebeelement mittels einer Zustelleinrichtung zum Zustellen des Behältnisses antreibbar gelagert ist. Hierdurch kann eine Verlagerung der Umlenkeinrichtung im Sinne der Erfindung aus einer Ausgangsposition heraus baulich sehr einfach realisiert werden.

Ferner ist es außergewöhnlich vorteilhaft, wenn die Vorrichtung eine Linearführungseinheit für die Umlenkeinrichtung mit einem daran translatorisch geführten Schiebeelement zum Verlagern der Umlenkeinrichtung aufweist, welche eine schwerkraftbetriebene Rückstelleinrichtung zum Rückstellen einer aus einer Ausgangsposition heraus verlagerten Umlenkeinrichtung aufweist. Aufgrund der schwerkraftbetriebenen Rückstelleinrichtung werden vorteilhafter Weise keine speziellen Kupplungselemente beispielsweise zwischen der Umlenkeinrichtung und der Trageeinrichtung erforderlich. In der Regel genügt es, wenn die Trageeinrichtung konstruktiv einfach an der Umlenkeinrichtung zur Anlage gebracht wird und die Umlenkeinrichtung dann einfach von der Trageinrichtung vorwärts bzw. hochwärts geschoben wird.

Insofern ist es vorteilhaft, wenn die Umlenkeinrichtung oder die Halteeinrichtung hierfür einen Anlagebereich zum temporären Anlegen der Trageeinrichtung zum Tragen des Behältnisses aufweist.

Es wäre auch denkbar, dass die Umlenkeinrichtung gleichzeitig auch als Halte- bzw. Greifeinrichtung zum Halten der Behältnisse dient, etwa, wenn die Umlenkeinrichtung selbst klammerartig ausgeführt ist.

Um die Umlenkeinrichtung an der Ausgangposition für einen neuen Sterilisationsvorgang mit geringem Aufwand sicher positionieren zu können, ist es vorteilhaft, wenn die Halteeinrichtung ein Auflagerelement aufweist, auf welchem ein Haltearmelement zum Halten der Umlenkeinrichtung auflegbar ist. Das Auflagerelement bildet hierbei vorteilhaft einen unterseitigen Anschlag an der Linearführungseinheit aus.

Da speziell in der Getränkeindustrie immer ein großes Bedürfnis an sicher sterilisierten Behältnissen vorhanden ist, wird die Aufgabe der Erfindung auch von einer Getränkeabfüllanlage und/oder einer Getränkebehälterherstellanlage mit einer Vorrichtung zum Umformen von Kunststoffvorformlingen zu Kunststoffbehältnissen gelöst, welche sich durch vorliegende Vorrichtung zum Sterilisieren von Behältnissen auszeichnet.

Nicht nur beim Handhaben von Getränken, welche gegenüber Keimen sehr empfindlich reagieren, sind oftmals ein Reinraum bzw. auch mehrere Reinräume vorgesehen, in welchen die Behälter unter aseptischen Bedingungen behandelt werden. Insofern ist es vorteilhaft, wenn die vorliegende Vorrichtung zum Sterilisieren von Behältnissen ebenfalls in einem derartigen Reinraum angeordnet ist.

Darüber hinaus betrifft die Erfindung noch eine Verwendung einer Zustell- und/oder Trageeinrichtung eines Behältnisses zum translatorischen Verschieben einer Umlenkeinrichtung zum Umlenken von durch eine Erzeugungseinrichtung erzeugten Ladungsträgern gegenüber einer Leiteinrichtung zum Leiten der Ladungsträgern einer Vorrichtung zum Sterilisieren des Behältnisses an einer Getränkeabfüllanlage und/oder einer Getränkebehälterherstellanlage mit einer Vorrichtung zum Umformen von Kunststoffvorformlingen zu Kunststoffbehältnissen. Vorteilhafter Weise können hierdurch insbesondere herkömmliche Vorrichtungen zum Sterilisieren eines Behältnisses zum Aufnehmen von Getränken oder dergleichen wesentlich kompakter gebaut werden.

Weitere Vorteile, Ziele und Eigenschaften vorliegender Erfindung werden anhand anliegender Zeichnung und nachfolgender Beschreibung erläutert, in welchen beispielhaft Vorrichtungen zum Sterilisieren eines Behältnisses mit einer gegenüber einer Leiteinrichtung für Ladungsträger und auch vorteilhaft gegenüber einer Trageeinrichtung des Behältnisses verlagerbaren Umlenkeinrichtung zum Umlenken der aus der Leiteinrichtung austretenden Ladungsträger auf einen außenliegenden Mündungsbereich des Behältnisses dargestellt und beschrieben sind. Die Zeichnungen zeigen:
- Figur 1: schematisch eine perspektivische Teilansicht eines ersten Ausführungsbeispiels einer Vorrichtung zum Sterilisieren eines Behältnisses umfassend eine Linearführungseinheit mit einem daran translatorisch geführten Schiebeschlitten zum Verlagern einer Umlenkeinrichtung zum Umlenken von Ladungsträgern, wobei die Linearführungseinheit im Wesentlichen parallel und radial neben einer Leiteinrichtung für die Ladungsträger und beabstandet von einer Trageeinrichtung des Behältnisses angeordnet ist;
- Figur 2: schematisch eine teilweise geschnittene Seitenansicht der Vorrichtung aus der Figur 1 mit in die Umlenkvorrichtung hinein verlagertem Mündungsbereich des Behältnisses während einer Außensterilisationsphase;
- Figur 3: schematisch weitere perspektivische Teilansicht der Vorrichtung aus den Figuren 1 und 2 mit der in das Behältnis hinein verlagerten Leiteinrichtung;
- Figur 4: schematisch eine weitere teilweise geschnittene Seitenansicht der Vorrichtung aus den Figuren 1 bis 3 während einer Innensterilisationsphase;
- Figur 5: schematisch eine perspektivische Teilansicht eines weiteren Ausführungsbeispiels einer Vorrichtung zum Sterilisieren eines Behältnisses umfassend eine Linearführungseinheit mit einem daran translatorisch geführten Schiebeschlitten zum Verlagern einer Umlenkeinrichtung zum Umlenken von Ladungsträgern, wobei die Linearführungseinheit eine Trageeinrichtung zum Tragen des Behältnisses umfasst;
- Figur 6: schematisch eine weitere perspektivische Teilansicht der Vorrichtung aus der Figur 5;
- Figur 7: schematisch eine Ansicht eines zweigeteilten Umlenkschirmelementes einer alternativen Umlenkeinrichtung für die Vorrichtung aus den Figuren 1 bis 4;
- Figur 8: schematisch eine perspektivische Teilansicht einer Getränkebehälterherstellanlage im Bereich der Vorrichtung 1 aus den Figuren 1 bis 4 und
- Figur 9: schematisch eine Aufsicht der Getränkebehälterherstellanlage aus der Figur 8 zum Herstellen von Kunststoffbehältnissen mit einer in einem Reinraum angeordneten Vorrichtung zum Umformen von Kunststoffvorformlingen zu Kunststoffbehältnissen und mit einer in dem Reinraum ebenfalls angeordneten Vorrichtung aus den Figuren 1 bis 4.

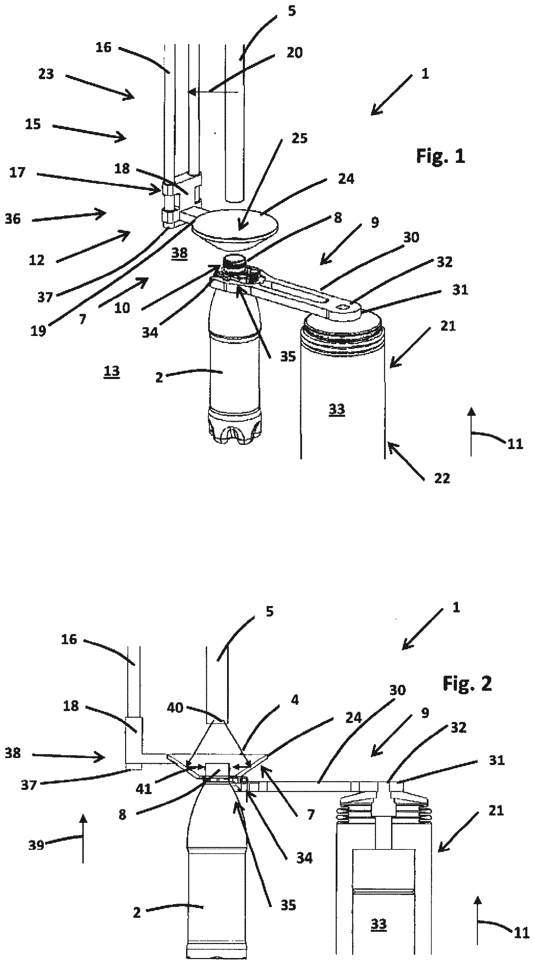

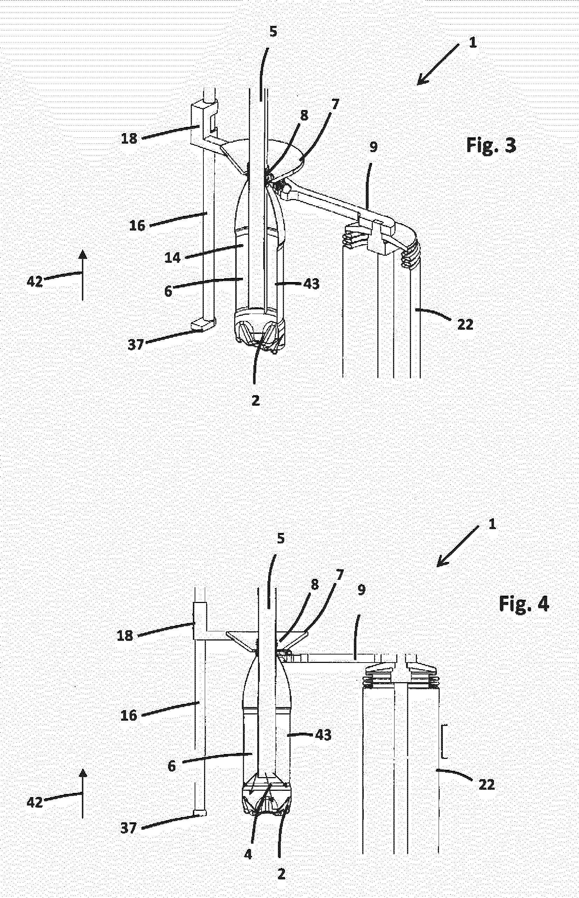

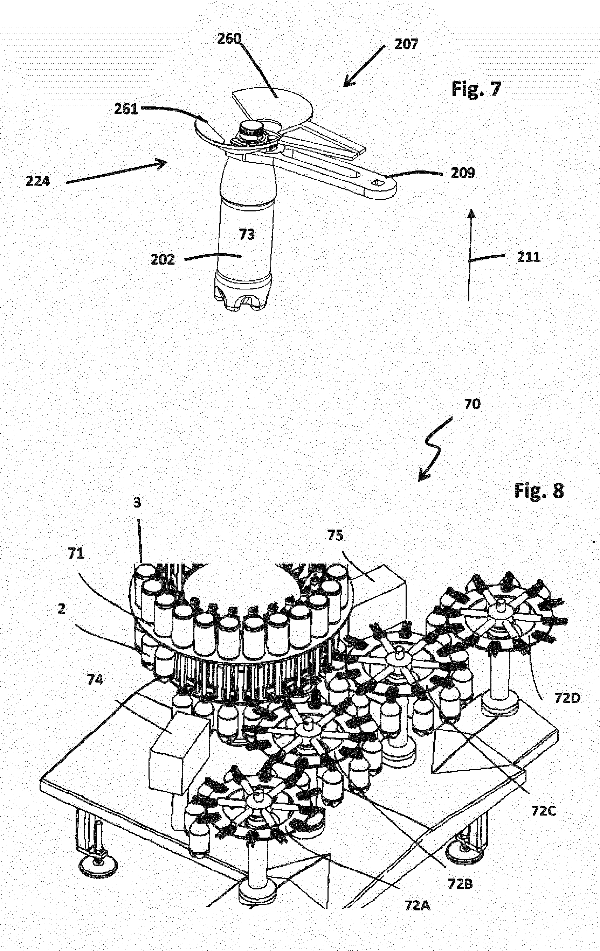

Das in den Figuren 1 bis 4 gezeigte erste Ausführungsbeispiel einer Vorrichtung 1 zum Sterilisieren eines Behältnisses 2 weist im Wesentlichen eine Erzeugungseinrichtung 3 (hier nicht gezeigt, siehe aber Figur 8) zum Erzeugen von Ladungsträgern 4 (siehe Figur 2), eine in das Behältnis 2 einbringbare Leiteinrichtung (Strahlfinger) 5 zum Beschleunigen und Leiten der Ladungsträger 4 auf eine Innenwandung 6 (siehe Figuren 3 und 4) sowie eine Umlenkeinrichtung 7 zum Umlenken der aus der Leiteinrichtung 5 austretenden Ladungsträger 4 auf einen außenliegenden Mündungsbereich 8 des Behältnisses 2 auf.

Die Umlenkeinrichtung 7 ist hierbei sowohl gegenüber der Leiteinrichtung 5 als auch gegenüber einer Trageeinrichtung 9 zum Tragen des Behältnisses 2 an einer Mündung 10 mittels einer einfachen Zustellbewegung 11 des Behältnisses 2 in dessen Längsrichtung verlagerbar ist.

Die Vorrichtung 1 weist hierzu eine Halteeinrichtung 12 für die Umlenkeinrichtung 7 auf, an welcher die Umlenkeinrichtung 7 mittels der Zustellbewegung 11 des Behältnisses 2 gegenüber der Leiteinrichtung 5 translatorisch verlagerbar angeordnet ist. Darüber hinaus ist die Trageeinrichtung 9 gegenüber der Umlenkeinrichtung 7 ebenfalls mittels der Zustellbewegung 11 des Behältnisses 2 translatorisch verlagerbar angeordnet. Hierdurch ergibt sich insgesamt ein besonders einfaches Zusammenspiel der vorstehend genannten Komponenten, sodass das Behältnis 2 zumindest an kritischen Bereichen sowohl außen 13 als auch innen 14 (vgl. Fig. 3) konstruktiv besonders einfach sterilisiert werden kann.

Die Halteeinrichtung 12 umfasst in diesem ersten Ausführungsbeispiel eine Linearführungseinheit 15 aus einer Doppelstangentraverse 16 und einem daran translatorisch geführten Schiebelement 17, welches hier als Schiebeschlitten 18 ausgeführt ist. Von dem Schiebeschlitten 18 steht ein Haltearm 19 zum Halten der Umlenkeinrichtung 7 (senkrecht) ab, sodass die Umlenkeinrichtung 7 ausreichend beabstandet von der Doppelstangentraverse 16 geführt werden kann.

Die Linearführungseinheit 15 ist im Wesentlichen parallel und radial 20 beabstandet neben der Leiteinrichtung 5 platziert, sodass sie problemlos die Umlenkeinrichtung 7 bis über die Leiteinrichtung 5 verschieben kann.

Insofern ist die Umlenkeinrichtung 7 bzw. das Schiebeelement 17 konstruktiv einfach auch mittels einer herkömmlichen Zustelleinrichtung 21 zum Zustellen des Behältnisses 2 antreibbar. Die Zustelleinrichtung 21 ist hier von einem bekannten Behälterhubelement 22 realisiert, sodass sie mit bekannten Bauteilen kostengünstig bereitgestellt werden kann. Es kommen hier beispielsweise elektronische, pneumatische oder hydraulische Antriebe in Betracht, denkbar wäre jedoch auch die Verwendung von Führungskurven.

Eine Rückstellbewegung der Umlenkeinrichtung 7, welche der Zustellbewegung 11 entgegengesetzt gerichtet ist, kann vorliegend besonders einfach mittels der auf die Umlenkeinrichtung 7, den Schiebeschlitten 18 und den Haltearm 19 wirkenden Schwerkraft erzielt werden. Insofern zeichnet sich die vorliegende Halteeinrichtung 12 auch durch eine implizierte schwerkraftbetriebene Rückstelleinrichtung 23 aus.

Die Umlenkeinrichtung 7 zeichnet sich in diesem Ausführungsbeispiel speziell durch ein konisch zulaufendes Umlenkschirmelement 24 mit einer zentrisch angeordneten Materialausnehmung bzw. Öffnung 25 aus. Die zentrisch angeordnete Materialausnehmung 25 hat hierbei einen derartigen Durchmesser, dass in ihr die Mündung 10 des Behältnisses 2 platziert werden kann, wenn das Behältnis 2 von unten gemäß der Zustellbewegung 11 an die Umlenkeinrichtung 7 herangeführt wird.

Die Trageeinrichtung 9 ist im Wesentlichen aus dem Stand der Technik bekannt. Sie weist einen Tragarm 30 auf, der mit einem ersten Tragarmende 31 an einem oberen Kolbenende 32 einer Zylindereinheit 33 der Zustelleinrichtung 21 befestigt ist. An seinem anderen Tragarmende 34 ist eine Aufnahme 35 zum Aufnehmen des Behältnisses 2 vorgesehen, in welche das Behältnis 2 mit seiner Mündung 10 eingehängt werden kann.

Zum Sterilisieren des Behältnisses 2 wird das Behältnis 2 gemäß der Darstellung nach der Figur 1 in die Aufnahme 35 der Trageeinrichtung 9 platziert. Hierbei liegt die Umlenkeinrichtung 7 noch in ihrer Ausgangposition 36 auf einen Anschlag 37 der Halteeinrichtung 12 am unteren Ende 38 der Doppelstangentraverse 16 auf. Die Erzeugungseinrichtung 3 zum Erzeugen der Ladungsträger 4 ist hier vorzugsweise noch inaktiv, kann jedoch auch aktiv bleiben.

Wie gemäß der Darstellung nach der Figur 2 gut zu entnehmen ist, ist das Behältnis 2 mittels der Zustelleinrichtung 21 in Richtung der Zustellbewegung 11 bereits um einen ersten Wegabschnitt 39 angehoben, bis die Mündung 10 des Behältnisses 2 durch die Materialausnehmung 25 hindurch bis innerhalb der Umlenkeinrichtung 7 hinein verlagert ist. Die Erzeugungseinrichtung 3 ist mittlerweile aktiviert und es werden Ladungsträger 4 durch die Leiteinrichtung 5 beschleunigt, die an einer unterseitigen Austrittsöffnung 40 aus der Leiteinrichtung 5 auch nach radial außen heraustreten und somit auf die Umlenkeinrichtung 7 geleitet werden. Die auf das Umlenkschirmelement 24 auftreffenden Ladungsträger 4 werden durch das Umlenkschirmelement 24 radial nach innen umgelenkt und treffen im Zuge dessen auf den außenliegenden Mündungsbereich 8 des Behältnisses 2, da sich die Mündung 10 des Behältnisses 2 im Strahlengang 41 (nur exemplarisch beziffert) der jeweiligen Ladungsträger 4 befindet. Hierdurch wird mit Hilfe der Ladungsträger 4 der außenliegende Mündungsbereich 8 sterilisiert.

Gemäß den Darstellungen der Figuren 3 und 4 ist die Umlenkeinrichtung 7 gemeinsam mit dem Behältnis 2 um einen weiteren Wegabschnitt 42 der translatorischen Zustellbewegung 11 in Richtung Leiteinrichtung 5 verlagert worden. Hierbei ist die Leiteinrichtung 5 durch die Umlenkeinrichtung 7 hindurch bis in das Volumen 43 des Behältnisses 2 eingetaucht, sodass nunmehr die Innenwandung 6 mit Hilfe der aus der Austrittsöffnung 40 austretenden Ladungsträger 4 sterilisiert wird. Insofern ist auch die Wandung des Mündungsbereiches 8 des Behälters 2 zumindest teilweise zwischen der Umlenkeinrichtung 7 und der Leiteinrichtung 5 angeordnet, wenn die Leiteinrichtung 5 in das Behältnis 2 eingebracht ist. Hierbei ist der Schiebeschlitten 18 von dem Anschlag 37 abgehoben und entlang der Doppelstangentraverse 16 in Richtung der Zustellbewegung 11 nach oben verlagert.

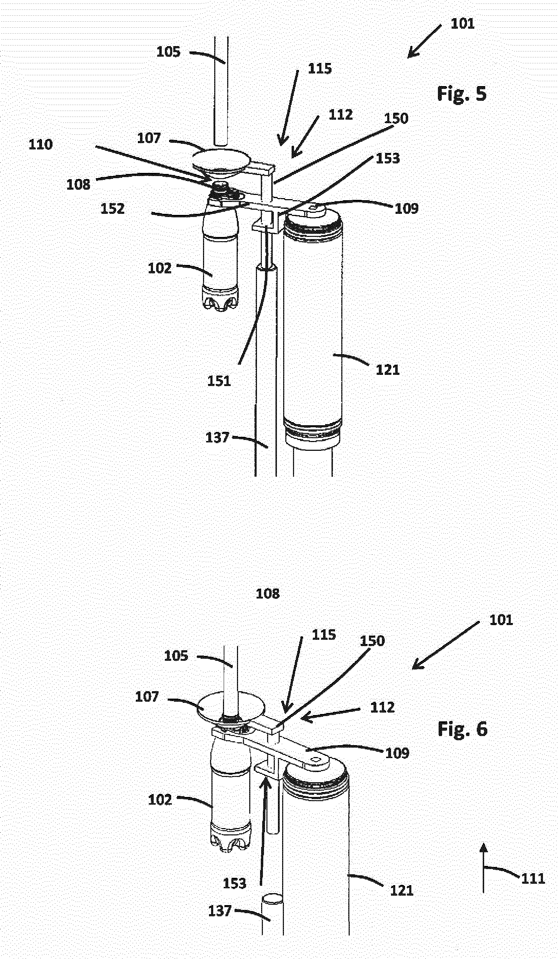

Das in den Figuren 5 und 6 gezeigte weitere Ausführungsbeispiel einer weiteren Vorrichtung 101 zum Sterilisieren eines Behältnisses 102 zeichnet sich ebenfalls im Wesentlichen durch eine Erzeugungseinrichtung 3 (siehe Figur 8) zum Erzeugen von Ladungsträgern 4 (siehe Figur 2), durch eine in das Behältnis 102 einbringbare Leiteinrichtung 105 zum Beschleunigen und Leiten der Ladungsträger 4 auf eine Innenwandung 6 (siehe Figuren 3 und 4) sowie durch eine Umlenkeinrichtung 107 zum Umlenken der aus der Leiteinrichtung 105 austretenden Ladungsträger 4 auf einen außenliegenden Mündungsbereich 108 einer Mündung 110 des Behältnisses 102 aus. Insofern werden nur die wesentlichen Unterschiede zum ersten Ausführungsbeispiel erläutert, um Wiederholungen zu vermeiden.

Die in den Figuren 5 und 6 gezeigte Vorrichtung 101 hat jedoch eine etwas anders gestaltete Halteeinrichtung 112 für die Umlenkeinrichtung 107, bei welcher eine Linearführungseinheit 115 eine Trageeinrichtung 109 des Behältnisses 102 vorteilhafter Weise sogleich umfasst. Die Linearführungseinheit 115 besitzt hierbei einen L-förmig gewinkelten Haltearm 150 zum Halten der Umlenkeinrichtung 107, welcher in einem in der Trageinrichtung 109 integrierten Doppellagerauge 151 verschieblich gelagert ist und welcher in einer Ausgangsposition der Umlenkeinrichtung 107 auf einem unterseitig angeordneten Anschlag 137 aufsitzt. Zum Realisieren des Doppellagerauges 151 ist ein Zwischenbereich 152 der Trageinrichtung 109 zwischen einer Zustelleinrichtung 121 und der Leiteinrichtung 105 als Gabelelement 153 ausgestaltet.

Verfährt nun die Trageeinrichtung 109 in Richtung der Zustellbewegung 111 kann zuerst die Mündung 110 des an der Trageeinrichtung 109 aufgehängten Behältnisses 102 bis in die Umlenkeinrichtung 107 hinein verlagert werden, wie vorstehend bereits hinsichtlich des ersten Ausführungsbeispiels beschrieben, da die Trageeinrichtung 109 verschieblich gegenüber der Halteeinrichtung 112 der Umlenkeinrichtung 107 gelagert ist. Hierbei kann der außenliegende Mündungsbereich 108 des Behältnisses 102 mit Hilfe der Ladungsträger sterilisiert werden. Wird die Trageeinrichtung 109 weiter gemäß der Zustellbewegung 111 verlagert, nimmt sie die Umlenkeinrichtung 107 hochwärts mit, wodurch sich die Leiteinrichtung 105 in das Behältnis 102 hinein verlagert. Hierbei kann das Behältnis 102 auch innen sterilisiert werden.

Die in der Figur 7 gezeigte alternative Umlenkeinrichtung 207 weist ein zweigeteiltes Umlenkschirmelement 224 auf, welches sich durch eine erste Schirmhälfte 260 und eine zweite Schirmhälfte 261 auszeichnet, welche hinsichtlich einer Zustellbewegung 211 eines an einer Trageeinrichtung 209 befindlichen Behältnisses 202 radial nach außen schwenkbar sind. Hierdurch können bedarfsweise alternative Zuführwege der Umlenkeinrichtung 207 bereitgestellt werden. Beispielsweise wird das zweigeteilte Umlenkschirmelement 224 bei einer Behältnisübergabe aufgeklappt, um das Behältnis 202 vorteilhafter übergeben zu können. Anschließend wird die Umlenkeinrichtung 207 geschlossen, um ein möglichst einheitliches Entkeimungsergebnis zu bekommen.

Die in der Figur 8 ausschnittsweise gezeigte Getränkebehälterherstellanlage 70 umfasst ein Sterilisationsrad 71 mit einer Vielzahl an Vorrichtungen 1 zum Sterilisieren von Behältnissen 2, wie sie vorstehend beispielhaft anhand der in den Figuren 1 bis 4 gezeigten Vorrichtung 1 beschrieben sind. Hierdurch kann eine Innenwand- und Mündungsbereichssterilisation vorteilhaft vorgenommen werden.

Die einzelnen Behältnisse 2 können dem Sterilisationsrad 71 mittels Transportsterne 72A bis 72C vorteilhaft zugeführt und von dem Sterilisationsrad 71 ebenso vorteilhaft wieder abgeführt werden. Zusätzlich können die großflächigeren Außenflächen 73 der Behältnisse 2 durch eine links dargestellte Außensterilisationsvorrichtung 74 vorgenommen werden, wobei die Behältnisse 2 bevorzugt in diesem Bereich um beispielsweise 180° bezüglich ihrer Längsachse gedreht werden können. Anschließend werden die Behältnisse 2 einzeln angehoben, um eine Innenwand- und Mündungsbereichssterilisation durchzuführen, wie oben bereits erläutert. Danach werden die Behältnisse 2 wieder abgesenkt und es wird abermals der übrigen Außenumfang mit einer rechten Außensterilisationsvorrichtung 75 sterilisiert, wobei hierzu bevorzugt die Behältnisse 2 um weitere 180°gedreht werden.

Auch wäre es möglich, die Behältnisse 2 während der Innenwand- und Mündungsbereichssterilisation zu drehen. Vorzugsweise sind daher entsprechende Dreheinrichtungen (hier nicht explizit beziffert) derart aufeinander abgestimmt, dass die beiden Sterilisationsvorrichtungen 74 und 75 eine großflächige Außensterilisation der Behältnisse 2 vornehmen können. Damit wird hier vorteilhafter Weise nur ein Sterilisationsrad 71 für die vollständige Außen- und Innensterilisation verwendet. Insbesondere durch das Vorsehen der beiden Außensterilisationsvorrichtungen 74 und 75 kann eine für die Außensterilisation nötige Drehgeschwindigkeit der Behältnisse 2 reduziert werden. Die in der Figur 8 gezeigte Anordnung erlaubt damit eine höhere Platzeinsparung. Vorteilhafter Weise könnte durch die vorliegende Erfindung auch sehr gut auf die beiden zusätzlichen Außensterilisationsvorrichtungen 74 und 75 verzichtet werden.

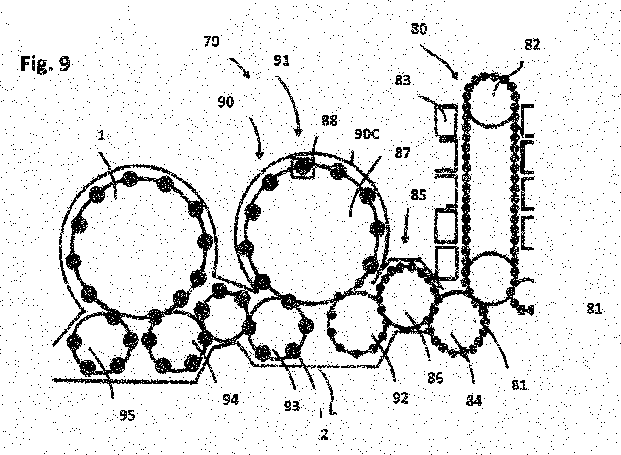

Wie gemäß den Darstellungen der Figuren 9 und 10 entnehmbar ist, weist die Getränkebehälterherstellanlage 70 noch eine Heizeinrichtung 80 auf, in der Kunststoffvorformlinge 81 erwärmt werden. Hierbei werden die Kunststoffvorformlinge 81 mittels einer Transporteinrichtung 82, wie hier einer umlaufenden Kette, durch die Heizeinrichtung 80 geführt und dabei mit einer Vielzahl von Heizelementen 83 erwärmt. An die Heizeinrichtung 80 schließt sich eine Übergabeeinheit 84 an, welche die Kunststoffvorformlinge 81 an eine vorgelagerte Sterilisationseinrichtung 85 übergibt. Diese vorgelagerte Sterilisationseinrichtung 85 weist ein Transportrad 86 auf und an diesem Transportrad 86 oder auch stationär können spezielle Sterilisationselemente (hier nicht gezeigt) angeordnet sein. In diesem Bereich ist beispielsweise eine Sterilisation durch Wasserstoffperoxidgas oder auch durch elektromagnetische bzw. UV-Strahlung möglich. Insbesondere wird in diesem Bereich eine Innensterilisation der Kunststoffvorformlinge 81 durchgeführt.

Das Bezugszeichen 90 bezeichnet in seiner Gesamtheit einen Reinraum, dessen Außenbegrenzungen hier durch die punktierte Linie L angedeutet ist. An einer bevorzugten Ausführungsform ist der Reinraum 90 nicht nur im Bereich der Vorrichtung 91 zum Umformen von Kunststoffvorformlingen 81 zu Behältnissen 2 und der erfindungsgemäßen Vorrichtung 1 zum Sterilisieren der Behältnisse 2 angeordnet, sondern beginnt möglicherweise schon im Bereich der Heizeinrichtung 80, der vorgelagerten Sterilisationseinrichtung 85, der Kunststoffvorformlingzuführung und/oder der Kunststoffvorformlingherstellung und erstreckt sich bis in eine an die Vorrichtung 1 anschließenden hier nicht gezeigten Befüllungseinrichtung der Behältnisse 2. Man erkennt, dass dieser Reinraum 90 in dem Bereich der vorgelagerten Sterilisationseinheit 85 beginnt. In diesem Bereich können Schleuseneinrichtungen vorgesehen sein, um die Kunststoffvorformlinge 81 in den Reinraum 90 einzuführen, ohne dass dabei zu viel Gas aus dem Reinraum 90 strömt und so verloren geht.

Der Reinraum 90 ist, wie durch die gestrichelte Linie L angedeutet, an die Außengestalt der einzelnen Anlagenkomponenten angepasst. Auf diese Weise kann das Volumen des Reinraums 90 reduziert werden. Das Bezugszeichen 91 bezeichnet in ihrer Gesamtheit die Umformungsvorrichtung, bei der an einem weiteren Transportrad 87 eine Vielzahl von Blasstationen 88 angeordnet ist, wobei hier lediglich eine dieser Blasstationen 88 dargestellt ist. Mit diesen Blasstationen 88 werden die Kunststoffvorformlinge 81 zu Behältnissen 2 expandiert. Obwohl hier nicht detailliert gezeigt, befindet sich nicht der gesamte Bereich des Transporteinrichtung 87 innerhalb des Reinraums 90, sondern der Reinraum 90 bzw. Isolator ist gewissermaßen als Mini-Isolator innerhalb der gesamten Vorrichtung realisiert. So wäre es möglich, dass der Reinraum 90 zumindest im Bereich der Umformungsvorrichtung 91 kanalartig ausgeführt ist.

Das Bezugszeichen 92 bezieht sich auf eine Zuführeinrichtung, welche die Kunststoffvorformlinge 81 an die Umformungseinrichtung 91 übergibt und das Bezugszeichen 93 auf eine Abführeinrichtung, welche die hergestellten Kunststoffbehältnisse 2 von der Umformungsvorrichtung 1 abführt. Man erkennt, dass der Reinraum 90 in dem Bereich der Zuführeinrichtung 92 und der Abführeinrichtung 93 jeweils Ausnehmungen aufweist, welche diese Einrichtungen 92, 93 aufnehmen. Auf diese Weise kann in besonders vorteilhafter Weise eine Übergabe der Kunststoffvorformlinge 81 an die Umformungsvorrichtung 91 bzw. eine Übernahme der Kunststoffbehältnisse 2 von der Umformungsvorrichtung 91 erreicht werden.

Mit einer Übergabeeinheit 94 werden die expandierten Behältnisse 2 an die Vorrichtung 1 zum Sterilisieren übergeben und von dieser Vorrichtung 1 anschließend über eine weitere Übergabeeinheit 95 abgeführt. Dabei befindet sich auch die Vorrichtung 1 innerhalb des besagten Reinraums 90. Die Übergabeeinheiten 94 und 95 umfassen hierbei die zuvor erwähnten Transportsterne 72A, 72B, 72C und 72D. Auch im Falle der anschließenden Befüllungseinrichtung wäre es möglich, dass nicht die gesamte Befüllungseinrichtung mit beispielsweise einem Reservoir für ein Getränk vollständig innerhalb des Reinraums 90 angeordnet ist, sondern auch hier lediglich diejenigen Bereiche, in denen tatsächlich die Behältnisse geführt werden. Insoweit könnte auch die Befüllungseinrichtung in ähnlicher Weise aufgebaut sein wie die Vorrichtung 91 zum Umformen von Kunststoffvorformlingen 81. Der Reinraum 90 ist auch im Bereich der Vorrichtung 91 auf einen geringstmöglichen Bereich reduziert, nämlich im Wesentlichen auf die Blasstationen 88 selbst. Durch diese kleinbauende Gestaltung des Reinraums 90 ist es leichter und schneller möglich, einen Reinraum 90 überhaupt herzustellen und auch die Sterilhaltung in der Betriebsphase ist weniger aufwändig. Auch wird weniger Sterilluft benötigt, was zu kleineren Filteranlagen führt und auch die Gefahr von unkontrollierter Wirbelbildung wird reduziert.

In einer weiteren Ausführungsform ist die Vorrichtung zum Sterilisieren von Behältnissen einer Behälterumformungseinrichtung, in welcher Preformen zu Flaschen umgeformt werden, vorgelagert. Der Aufbau entspricht im Wesentlichen der oben beschriebenen Ausführungsform, wobei geringfügige konstruktive Änderungen durchgeführt werden können. Es werden dabei die Preformen innen und zusätzlich der Mündungsbereich außen sterilisiert.

Auf eine zusätzliche Vorrichtung zur Entkeimung der vollständigen Außenwandung der Preformen könnte hierbei verzichtet werden.

### Bezugszeichenliste

- 1: Vorrichtung zum Sterilisieren
- 2: Behältnis
- 3: Erzeugungseinrichtung
- 4: Ladungsträger
- 5: Leiteinrichtung
- 6: Innenwandung
- 7: Umlenkeinrichtung
- 8: Mündungsbereich
- 9: Trageeinrichtung
- 10: Mündung
- 11: Zustellbewegung
- 12: Halteeinrichtung
- 13: außen
- 14: innen
- 15: Linearführungseinheit
- 16: Doppelstangentraverse
- 17: Schiebeelement
- 18: Schiebschlitten
- 19: Haltearm
- 20: radial
- 21: Zustelleinrichtung
- 22: Behälterhubelement
- 23: Rückstelleinrichtung
- 24: Umlenkschirmelement
- 25: Materialausnehmung
- 30: Tragarm
- 31: erstes Tragarmende
- 32: Kolbenende
- 33: Zylindereinheit
- 34: anderes Tragarmende
- 35: Aufnahme
- 36: Ausgangsposition
- 37: Anschlag
- 38: unteres Ende
- 39: erster Wegabschnitt
- 40: Austrittsöffnung
- 41: Strahlengang
- 42: weiterer Wegabschnitt
- 43: Volumen
- 70: Getränkebehälterherstellanlage
- 71: Sterilisationsrad
- 72A: Transportstern
- 72B: Transportstern
- 72C: Transportstern
- 72D: Transportstern
- 73: Außenflächen
- 74: erste Außensterilisationsvorrichtung
- 75: zweite Außensterilisationsvorrichtung
- 80: Heizeinrichtung
- 81: Kunststoffvorformlinge
- 82: Transporteinrichtung
- 83: Heizelemente
- 84: Übergabeeinheit
- 85: vorgelagerte Sterilisationseinrichtung
- 86: Transportrad
- 87: weiteres Transportrad
- 88: Blasstationen
- 89: Boden
- 90: Reinraum
- 91: Vorrichtung zum Umformen
- 92: Zuführeinrichtung
- 93: Abführeinrichtung
- 94: Übergabeeinheit
- 95: weitere Übergabeeinheit
- 96: Träger
- 101: Vorrichtung zum Sterilisieren
- 102: Behältnis
- 105: Leiteinrichtung
- 107: Umlenkeinrichtung
- 108: Mündungsbereich
- 109: Trageeinrichtung
- 110: Mündung
- 111: Zustellbewegung
- 112: Halteeinrichtung
- 115: Linearführungseinheit
- 121: Zustelleinrichtung
- 124: Umlenkschirmelement
- 150: I-förmig gewinkelter Haltearm
- 151: Doppellagerauge
- 152: Zwischenbereich
- 153: Gabelelement
- 202: Behältnis
- 207: Umlenkeinrichtung
- 209: Trageeinrichtung
- 211: Zustellbewegung
- 224: zweigeteiltes Umlenkschirmelement
- 260: erste Schirmhälfte
- 261: zweite Schirmhälfte

## Patentansprüche

1. Vorrichtung (1; 101) zum Sterilisieren eines Behältnisses (2; 102; 202) mit einer Erzeugungseinrichtung (3) zum Erzeugen von Ladungsträgern (4) und mit einer in das Behältnis (2; 102; 202) einbringbaren Leiteinrichtung (5; 105) zum Leiten der Ladungsträger (4) auf eine Innenwandung (6) des Behältnisses (2; 102; 202), wobei eine Mündung (10; 110) des Behältnisses (2; 102; 202) mittels einer Trageeinrichtung (9; 109; 209) zum Tragen des Behältnisses (2; 102; 202) gegenüber der Leiteinrichtung (5; 105) positionierbar ist,
**dadurch gekennzeichnet, dass**
die Vorrichtung (1; 101) eine gegenüber der Leiteinrichtung (5; 105) verlagerbare Umlenkeinrichtung (7; 107; 207) zum Umlenken der aus der Leiteinrichtung (5; 105) austretenden Ladungsträger (4) auf einen außenliegenden Mündungsbereich (8; 108) des Behältnisses (2; 102; 202) aufweist.

2. Vorrichtung nach Anspruch 1,
**dadurch gekennzeichnet, dass**
die Umlenkeinrichtung wenigstens zeitweise eine Öffnung aufweist, von der ein Mündungsbereich des Behältnisses umgebbar ist.

3. Vorrichtung (1; 101) nach Anspruch 1,
**dadurch gekennzeichnet, dass**
die Vorrichtung (1; 101) eine Halteeinrichtung (12) für die Umlenkeinrichtung (7; 107; 207) aufweist, an welcher die Umlenkeinrichtung (7; 107; 207) mittels einer Relativbewegung (11; 111) des Behältnisses (2; 102; 202) gegenüber der Leiteinrichtung (5; 105) translatorisch verlagerbar angeordnet ist und/oder gegenüber welcher die Trageeinrichtung (9; 109; 209) mittels einer Relativbewegung (11; 111; 211) des Behältnisses (2; 102; 202) translatorisch verlagerbar angeordnet ist.

4. Vorrichtung (1; 101) nach Anspruch 1 oder 2,
**dadurch gekennzeichnet, dass**
die Vorrichtung (1; 101) eine Linearführungseinheit (15; 115) für die Umlenkeinrichtung (7) mit einem daran translatorisch geführten Schiebeelement (17) zum Verlagern der Umlenkeinrichtung (7; 107; 207) aufweist, wobei bevorzugt die Linearführungseinheit (15; 115) im Wesentlichen parallel und radial (20) neben der Leiteinrichtung (5; 105) und beabstandet von der Trageeinrichtung (9; 109; 209) des Behältnisses (2) angeordnet ist oder wobei die Linearführungseinheit (115) die Trageeinrichtung (9; 109; 209) des Behältnisses (2; 102; 202) umfasst.

5. Vorrichtung (1; 101) nach einem der Ansprüche 1 bis 3,
**dadurch gekennzeichnet, dass**
die Vorrichtung (1; 101) eine Linearführungseinheit (15; 115) für die Umlenkeinrichtung (7; 107; 207) mit einem daran translatorisch geführten Schiebeelement (17) zum Verlagern der Umlenkeinrichtung (7; 107; 207) aufweist, an welcher die Umlenkeinrichtung (7; 107; 207) bzw. das Schiebeelement (17) mittels einer Zustelleinrichtung (21; 121) zum Zustellen des Behältnisses (2; 102; 202) antreibbar gelagert ist.

6. Vorrichtung (1; 101) nach einem der Ansprüche 1 bis 4,
**dadurch gekennzeichnet, dass**
die Vorrichtung (1; 101) eine Linearführungseinheit (15; 115) für die Umlenkeinrichtung (7; 107; 207) mit einem daran translatorisch geführten Schiebeelement (17) zum Verlagern der Umlenkeinrichtung (7; 107; 207) aufweist, welche eine schwerkraftbetriebene Rückstelleinrichtung (23) zum Rückstellen einer aus einer Ausgangsposition (36) heraus verlagerten Umlenkeinrichtung (7; 107; 207) aufweist.

7. Vorrichtung (1; 101) nach einem der Ansprüche 1 bis 5,
**dadurch gekennzeichnet, dass**
bei in das Behältnis (2; 102; 202) eingebrachter Leiteinrichtung (5; 105) die Wandung des Mündungsbereiches (8) des Behältnisses (2; 102; 202) zumindest teilweise zwischen der Umlenkeinrichtung (7; 107; 207) und der Leiteinrichtung (5; 105) angeordnet ist.

8. Vorrichtung (1; 101) nach einem der Ansprüche 1 bis 6,
**dadurch gekennzeichnet, dass**
die Umlenkeinrichtung (7) ein konisch zulaufendes Umlenkschirmelement (24; 124; 224) aufweist, welches konzentrisch um die Leiteinrichtung (5; 105) anordenbar ist, wenn die Mündung (10; 110) des Behältnisses (2; 102; 202) zumindest teilweise innerhalb der Umlenkeinrichtung (7; 107; 207) angeordnet ist.

9. Vorrichtung (1; 101) nach einem der Ansprüche 1 bis 7,
**dadurch gekennzeichnet, dass**
die Umlenkeinrichtung (207) ein zweigeteiltes Umlenkschirmelement (224) umfasst.

10. Getränkeabfüllanlage und/oder Getränkebehälterherstellanlage (70) mit einer Vorrichtung (91) zum Umformen von Kunststoffvorformlingen (81) zu Kunststoffbehältnissen (2; 102; 202),
**gekennzeichnet durch,**
eine Vorrichtung (1; 101) zum Sterilisieren nach einem der vorhergehenden Ansprüche.

11. Verfahren zum Sterilisieren eines ein von einer Innenwandung (6) umgrenzten Volumen (43) aufweisenden Behältnisses (2; 102; 202), bei welchem zum Sterilisieren des Behältnisses (2; 102; 202) Ladungsträger (4) erzeugt und die erzeugten Ladungsträger (4) aus einer Austrittsöffnung (40) einer Leiteinrichtung (5; 105) zum Leiten der Ladungsträger (4) heraus in Richtung des Behältnisses (2; 102; 202) beschleunigt werden, um das Behältnis (2; 102; 202) mit Hilfe der Ladungsträger (4) zumindest teilweise zu sterilisieren, wobei das Behältnis (2; 102; 202) der Leiteinrichtung (5; 105) mittels einer translatorischen Relativbewegung (11; 111; 211) des Behältnisses (2; 102; 202) gegenüber der Leiteinrichtung (5; 105) zugestellt wird,
**dadurch gekennzeichnet, dass**
in einem Wegabschnitt (39) der translatorischen Relativbewegung (11; 111; 211) eine Mündung (10; 110) des Behältnisses (2; 102; 202) in eine Umlenkeinrichtung (7; 107; 207) zum Umlenken der aus der Leiteinrichtung (5; 105) austretenden Ladungsträger (4) zumindest teilweise hinein verlagert wird, sodass ein außenliegender Mündungsbereich (8; 108) des Behältnisses (2; 102; 202) in einen Strahlengang (41) der umgelenkten Ladungsträger (4) hinein verlagert ist und hierdurch mit Hilfe der Ladungsträger (4) sterilisiert wird.

12. Verfahren nach Anspruch 11,
**dadurch gekennzeichnet, dass**
in einem weiteren Wegabschnitt (42) der translatorischen Relativbewegung (11; 111; 211) die Umlenkeinrichtung (7; 107; 207) gemeinsam mit dem Behältnis (2; 102; 202) derart in Richtung Leiteinrichtung (5; 105) verlagert werden, dass die Leiteinrichtung (5; 105) durch die Umlenkeinrichtung (7; 107; 207) hindurch in das Volumen (43) des Behältnisses (2; 102; 202) oder aus diesem Volumen heraus verlagert wird, sodass die Innenwandung (6) des Behältnisses (2; 102; 202) mit Hilfe der Ladungsträger (4) sterilisiert wird.

## Claims

1. An apparatus (1; 101) for the sterilization of a container (2; 102; 202) with a production device (3) for producing charge carriers (4) and with a guiding device (5; 105) capable of being introduced into the container (2; 102; 202) for guiding the charge carriers (4) to an inner wall (6) of the container (2; 102; 202), wherein an aperture (10; 110) of the container (2; 102; 202) is capable of being positioned with respect to the guiding device (5; 105) by means of a carrier device (9; 109; 209) for carrying the container (2; 102; 202),
**characterized in that**
the apparatus (1; 101) has a deflecting device (7; 107; 207) displaceable with respect to the guiding device (5; 105) for deflecting the charge carriers (4) issuing from the guiding device (5; 105) to an aperture area (8; 108) of the container (2; 102; 202) situated on the outside.

2. An apparatus according to claim 1,
**characterized in that**
the deflecting device has an opening at least for a time, by which an aperture area of the container is capable of being surrounded.

3. An apparatus (1; 101) according to claim 1,
**characterized in that**
the apparatus (1; 101) has a holding device (12) for the deflecting device (7; 107; 207), on which the deflecting device (7; 107; 207) is arranged so as to be displaceable in a translational manner with respect to the guiding device (5; 105) by means of a relative movement (11; 111) of the container (2; 102; 202) and/or with respect to which the carrier device (9; 109; 209) is arranged so as to be displaceable in a translational manner by means of a relative movement (11; 111; 211) of the container (2; 102; 202).

4. An apparatus (1; 101) according to claim 1 or 2,
**characterized in that**
the apparatus (1; 101) has a linear guide unit (15; 115) for the deflecting device (7) with a slide element (17) guided in a translational manner on it for the displacement of the deflecting device (7; 107; 207), wherein the linear guide unit (15; 115) is preferably arranged substantially parallel and radially (20) adjacent to the guiding device (5; 105) and at a distance from the carrier device (9; 109; 209) of the container (2) or wherein the linear guide unit (115) comprises the carrier unit (9; 109; 209) of the container (2; 102; 202).

5. An apparatus (1; 101) according to any one of claims 1 to 3,
**characterized in that**
the apparatus (1; 101) has a linear guide unit (15; 115) for the deflecting device (7; 107; 207) with a slide element (17) guided in a translational manner thereon for the displacement of the deflecting device (7; 107; 207), on which linear guide unit the deflecting device (7; 107; 207) or the slide element (17) is mounted in a manner capable of being driven by means of a feed device (21; 121) for feeding the container (2; 102; 202).

6. An apparatus (1; 101) according to any one of claims 1 to 4,
**characterized in that**
the apparatus (1; 101) has a linear guide unit (15; 115) for the deflecting device (7; 107; 207) with a slide element (17) guided in a translational manner thereon for the displacement of the deflecting device (7; 107; 207), which has a returning device (23) operated by gravity for returning a deflecting device (7; 107; 207) displaced out of a starting position (36).

7. An apparatus (1; 101) according to any one of claims 1 to 5,
**characterized in that**
the wall of the aperture area (8) of the container (2; 102; 202) is arranged at least in part between the deflecting device (7; 107; 207) and the guiding device (5; 105) when the guiding device (5; 105) is inserted into the container (2; 102; 202).

8. An apparatus (1; 101) according to any one of claims 1 to 6,
**characterized in that**
the deflecting device (7) has a deflection screen element (24; 124; 224) which converges in a conical manner and which is capable of being arranged concentrically around the guiding device (5; 105) if the aperture (10; 110) of the container (2; 102; 202) is arranged at least in part inside the deflecting device (7; 107; 207).

9. An apparatus (1; 101) according to any one of claims 1 to 7,
**characterized in that**
the deflecting device (207) comprises a deflection screen element (224) divided into two.

10. A beverage filling plant and/or a beverage container production plant (70) with an apparatus (91) for shaping plastics material pre-forms (81) into plastics material containers (2; 102; 202),
**characterized by**
an apparatus (1; 101) for sterilization according to any one of the preceding claims.

11. A method of sterilizing a container (2; 102; 202) having a volume (43) bounded by an inner wall (6), in which charge carriers (4) are produced for the sterilization of the container (2; 102; 202) and the charge carriers (4) produced are accelerated in the direction of the container (2; 102; 202) out of an outlet opening (40) of a guiding device (5; 105) for guiding the charge carriers (4), in order to sterilize the container (2; 102; 202) with the aid of the charge carriers (4) at least in part, wherein the container (2; 102; 202) is fed to the guiding device (5; 105) by means of a translational relative movement (11; 111; 211) of the container (2; 102; 202) with respect to the guiding device (5; 105),
**characterized in that**
in a stretch (39) of the path of the translational relative movement (11; 111; 211) an aperture (10; 110) of the container (2; 102; 202) is displaced at least in part into a deflecting device (7; 107; 207) for deflecting the charge carriers (4) issuing from the guiding device (5; 105), so that an aperture area (8; 108) of the container (2; 102; 202) situated on the outside is displaced into a beam path (41) of the deflected charge carriers (4) and, as a result, is sterilized with the aid of the charge carriers (4).

12. A method according to claim 11,
**characterized in that**
in a further stretch (42) of the path of the translational relative movement (11; 111; 211) the deflecting device (7; 107; 207) is displaced jointly with the container (2; 102; 202) in the direction of the guiding device (5; 105) in such a way that the guiding device (5; 105) is displaced through the deflecting device (7; 107; 207) into the volume (43) of the container (2; 102; 202) or out of this volume, so that the inner wall (6) of the container (2; 102; 202) is sterilized with the aid of the charge carriers (4).

## Revendications

1. Dispositif (1 ; 101) de stérilisation d'un récipient (2 ; 102 ; 202) avec un équipement de production (3) pour la production de porteurs de charge (4) et avec un équipement de guidage (5 ; 105) pouvant être introduit dans le récipient (2 ; 102 ; 202) pour guider les porteurs de charge (4) sur une paroi intérieure (6) du récipient (2 ; 102 ; 202), dans lequel une embouchure (10 ; 110) du récipient (2 ; 102 ; 202) peut être positionnée au moyen d'un équipement porteur (9 ; 109; 209) pour porter le récipient (2 ; 102; 202) par rapport à l'équipement de guidage (5 ; 105),
**caractérisé en ce que**
le dispositif (1 ; 101) présente un équipement déflecteur (7 ; 107 ; 207) déplaçable par rapport à l'équipement de guidage (5 ; 105) pour orienter les porteurs de charge (4) sortant de l'équipement de guidage (5 ; 105) sur une zone d'embouchure située à l'extérieur (8 ; 108) du récipient (2 ; 102 ; 202).

2. Dispositif selon la revendication 1,
**caractérisé en ce que**
l'équipement déflecteur présente au moins temporairement une ouverture de laquelle une zone d'embouchure du récipient peut être entourée.

3. Dispositif (1 ; 101) selon la revendication 1,
**caractérisé en ce que**
le dispositif (1 ; 101) présente un équipement de retenue (12) pour l'équipement déflecteur (7 ; 107 ; 207), au niveau duquel l'équipement déflecteur (7 ; 107; 207) est agencé de manière déplaçable en translation au moyen d'un mouvement relatif (11 ; 111) du récipient (2 ; 102 ; 202) par rapport à l'équipement de guidage (5 ; 105) et/ou par rapport auquel l'équipement porteur (9 ; 109; 209) est agencé de manière déplaçable en translation au moyen d'un mouvement relatif (11 ; 111 ; 211) du récipient (2 ; 102 ; 202).

4. Dispositif (1 ; 101) selon la revendication 1 ou 2,
**caractérisé en ce que**
le dispositif (1 ; 101) présente une unité de guidage linéaire (15 ; 115) pour l'équipement déflecteur (7) avec un élément coulissant (17) guidé par translation à son niveau pour déplacer l'équipement déflecteur (7 ; 107 ; 207), dans lequel de préférence l'unité de guidage linéaire (15 ; 115) est agencée de manière sensiblement parallèle et radiale (20) à côté de l'équipement de guidage (5 ; 105) et à distance de l'équipement porteur (9 ; 109 ; 209) du récipient (2) ou dans lequel l'unité de guidage linéaire (115) comprend l'équipement porteur (9 ; 109; 209) du récipient (2 ; 102 ; 202).

5. Dispositif (1 ; 101) selon l'une quelconque des revendications 1 à 3,
**caractérisé en ce que**
le dispositif (1 ; 101) présente une unité de guidage linéaire (15 ; 115) pour l'équipement déflecteur (7 ; 107; 207) avec un élément coulissant (17) guidé par translation à son niveau pour déplacer l'équipement déflecteur (7 ; 107; 207), au niveau de laquelle l'équipement déflecteur (7 ; 107 ; 207) ou l'élément coulissant (17) est logé de manière à pouvoir être entraîné au moyen d'un équipement d'amenée (21 ; 121) pour amener le récipient (2 ; 102 ; 202).

6. Dispositif (1 ; 101) selon l'une quelconque des revendications 1 à 4,
**caractérisé en ce que**
le dispositif (1 ; 101) présente une unité de guidage linéaire (15 ; 115) pour l'équipement déflecteur (7 ; 107; 207) avec un élément coulissant (17) guidé par translation à son niveau pour déplacer l'équipement déflecteur (7 ; 107 ; 207), laquelle présente un équipement de rappel fonctionnant par gravité (23) pour ramener un équipement déflecteur (7 ; 107 ; 207) déplacé hors d'une position de départ (36).

7. Dispositif (1 ; 101) selon l'une quelconque des revendications 1 à 5,
**caractérisé en ce que**
lorsque l'équipement de guidage (5 ; 105) est introduit dans le récipient (2 ; 102 ; 202), la paroi de la zone d'embouchure (8) du récipient (2 ; 102 ; 202) est agencée au moins en partie entre l'équipement déflecteur (7 ; 107 ; 207) et l'équipement de guidage (5 ; 105).

8. Dispositif (1 ; 101) selon l'une quelconque des revendications 1 à 6,
**caractérisé en ce que**
l'équipement déflecteur (7) présente un élément d'écran déflecteur conique (24 ; 124; 224), lequel peut être agencé de manière concentrique autour de l'équipement de guidage (5 ; 105) lorsque l'embouchure (10 ; 110) du récipient (2 ; 102; 202) est agencée au moins en partie à l'intérieur de l'équipement déflecteur (7 ; 107 ; 207).

9. Dispositif (1 ; 101) selon l'une quelconque des revendications 1 à 7,
**caractérisé en ce que**
l'équipement déflecteur (207) comprend un élément d'écran déflecteur (224) divisé en deux parties.

10. Installation de remplissage de boissons et/ou installation de fabrication de récipients de boissons (70) avec un dispositif (91) de transformation de préformes en plastique (81) en récipients en plastique (2 ; 102 ; 202),
**caractérisée par**
un dispositif (1 ; 101) de stérilisation selon l'une quelconque des revendications précédentes.

11. Procédé de stérilisation d'un récipient (2 ; 102; 202) contenant des volumes (43) limités par une paroi intérieure (6), dans lequel des porteurs de charge (4) sont produits pour la stérilisation du récipient (2 ; 102 ; 202) et les porteurs de charge (4) produits sont accélérés hors d'une ouverture de sortie (40) d'un équipement de guidage (5 ; 105) pour guider les porteurs de charge (4) en direction du récipient (2 ; 102 ; 202), afin de stériliser au moins en partie le récipient (2 ; 102 ; 202) à l'aide des porteurs de charge (4), dans lequel le récipient (2 ; 102 ; 202) est amené à l'équipement de guidage (5 ; 105) au moyen d'un mouvement relatif de translation (11 ; 111 ; 211) du récipient (2 ; 102; 202) par rapport à l'équipement de guidage (5 ; 105),
**caractérisé en ce que**
dans un tronçon (39) du mouvement relatif de translation (11 ; 111 ; 211), une embouchure (10 ; 110) du récipient (2 ; 102 ; 202) est déplacée au moins en partie dans un équipement déflecteur (7 ; 107 ; 207) pour orienter les porteurs de charge (4) sortant de l'équipement de guidage (5 ; 105), si bien qu'une zone d'embouchure située à l'extérieur (8 ; 108) du récipient (2 ; 102 ; 202) est déplacée dans une trajectoire de faisceau (41) des porteurs de charge orientés (4) et ainsi stérilisée à l'aide des porteurs de charge (4).

12. Procédé selon la revendication 11,
**caractérisé en ce que**
dans un autre tronçon (42) du mouvement relatif de translation (11 ; 111 ; 211), l'équipement déflecteur (7 ; 107 ; 207) est déplacé conjointement avec le récipient (2 ; 102; 202) en direction de l'équipement de guidage (5 ; 105) de telle manière que l'équipement de guidage (5 ; 105) est déplacé à travers l'équipement déflecteur (7 ; 107 ; 207) dans le volume (43) du récipient (2 ; 102 ; 202) ou hors de ce volume, si bien que la paroi intérieure (6) du récipient (2 ; 102 ; 202) est stérilisée à l'aide des porteurs de charge (4).
